# EUROPEAN PATENT SPECIFICATION

(11) **EP 4 275 456 B1**
(45) Date of publication and mention of the grant of the patent: **14.08.2024**
(21) Application number: 22700548.5
(22) Date of filing: 03.01.2022
(51) Int. Cl.: A61L 2/10, A61L 9/20, H05B 47/115

(54) **LUMINAIRE FOR EMITTING ULTRAVIOLET RADIATION**
LEUCHTE ZUR EMISSION VON ULTRAVIOLETTER STRAHLUNG
LUMINAIRE D'ÉMISSION DE RAYONNEMENT ULTRAVIOLET

(30) Priority: 08.01.2021 EP 21150659
(43) Date of publication of application: 15.11.2023
(73) Proprietor: Signify Holding B.V., 5656 AE Eindhoven (NL)
(72) Inventor: MAKKAI, Máté, 5656 AE Eindhoven (NL)
(74) Representative: Verweij, Petronella Daniëlle
(86) International application number: PCT/EP2022/050018
(87) International publication number: WO 2022/148724

(56) References cited:
- US-A1- 2009 218 512
- US-A1- 2014 131 595
- US-A1- 2018 193 501

## Description

### FIELD OF THE INVENTION

The present invention relates to a luminaire for emitting ultraviolet radiation, wherein the luminaire has a sensor to detect motion or presence of an object, such as a person. In particular the invention relates to a luminaire for emitting ultraviolet radiation according to claim 1. Preferred embodiments of the invention are defined by the dependent claims.

### BACKGROUND OF THE INVENTION

In medical facilities, disinfection of equipment, instruments, and other objects is important to reduce the transmission of pathogens and prevent the spread of illnesses between individuals. Given the recent developments, disinfection has become an increasingly important topic. Ultraviolet radiation sources are important candidates for disinfection and germicidal applications. Ultraviolet germicidal irradiation (UVGI) is a disinfection method that uses ultraviolet radiation, particularly short-wavelength ultraviolet radiation such as ultraviolet C or UV-C radiation, to kill or inactivate microorganisms by destroying nucleic acids and disrupting their DNA, leaving them unable to perform vital cellular functions. UVGI is used in a variety of applications, such as food, air, and water purification.

However, exposure to ultraviolet radiation, particularly short-wavelength ultraviolet radiation such as ultraviolet C or UV-C radiation, may be harmful and may provide damage to the eyes and skin of humans. The degree of damage from ultraviolet radiation, and thus to what extent the ultraviolet radiation is considered harmful, may be dependent on intensity, proximity, line of sight and time of exposure.

Accordingly, having ultraviolet radiation sources as part of a UVGI disinfection system in spaces where humans risk exposure to ultraviolet radiation is associated with danger. There may be a risk of damage to the eyes and/or skin of the people present in the vicinity of the UVGI disinfection system caused by the ultraviolet radiation. This may be solved by providing the people with proper safety equipment.

Another approach may be to use different sensors to trigger the ultraviolet radiation source based on the absence of people, that otherwise risk exposure to harmful ultraviolet radiation. One such approach is presented in US-2016/296650, wherein sensors are detecting if someone enters a room and wherein ultraviolet radiation is only provided when no person is in the room.

US-2014/131595 discloses a device, such as a toilet seat, for transmitting UV light over a broad area and for a long distance to inactivate microbes and non-microbial sources. The device has a plurality of UV light emitting sources embedded within the toilet seat and positioned near a side surface of the device. In operation, the embedded UV light emitting sources project UV light through the toilet seat to sanitize, disinfect and/or decontaminate the surface of the device and its surrounding areas, wherein the toilet seat acts as a single lens for the plurality of UV light emitting sources. The UV light emitting sources are powered by power source and they are operably linked to a control module, which in turn is operably associated with a detector for detecting an object or person so as to avoid and prevent unwanted exposure to UV light. The control module is arranged to receive input from the detector 4 and to send output to the UV light emitting sources. The controller, the detector, and the power source are located in a hub, which is disposed on or adjacent to the device.

US-2018/193501 discloses a hybrid germicidal irradiation apparatus with a fixture housing. The apparatus has a visible light emitter, a UV-C emitter, a near-UV emitter, a UV-C sensor, a near-UV sensor, a controller, a UV transmittable lens, a ranging sensor, and an occupant sensor. The apparatus can be installed in a ceiling, wherein the occupant sensor is affixed remotely to the apparatus, or wherein the occupant sensor is coupled to a face portion of the fixture housing.

It is desired to improve the safety, efficiency and/or performance of disinfection light sources.

### SUMMARY OF THE INVENTION

It is an object of the present invention to overcome at least some of the abovementioned problems.

According to a first aspect, a luminaire for emitting ultraviolet radiation is provided. The luminaire comprises an ultraviolet radiation source having a field of irradiation and an optical sensor having a field of view. The optical sensor is configured to detect motion or presence of an object in the field of view. The object may be a human being or an animal. The optical sensor is also configured to output a signal indicative of detected motion or presence of the object. The luminaire also comprises a control unit configured to control the ultraviolet radiation source based on the signal from the optical sensor. The control unit is connected to the ultraviolet radiation source and is configured to switch off the ultraviolet radiation source upon receipt of the signal indicative of motion or presence of the object. The luminaire further comprises an optical system configured to shape the field of irradiation of the ultraviolet radiation source and the field of view of the optical sensor. The ultraviolet radiation source and the optical sensor are both located upstream of the optical system such that the field of irradiation of the ultraviolet radiation source falls within the field of view of the optical sensor downstream of the optical system.

With this design, the harmful ultraviolet radiation from the ultraviolet radiation source will be interrupted as soon as a person or animal enters the field of view of the optical sensor, which may fully cover the field of irradiation of the ultraviolet radiation source, i.e. the field of view of the optical sensor may be substantially the same as or larger than the field of irradiation of the ultraviolet radiation source. This allows for a compact and reliable solution wherein the ultraviolet radiation is switched off only when a person or animal is at risk of entering the field of irradiation of the ultraviolet radiation source. Hence, it may be possible to reside in the vicinity of the ultraviolet radiation source when the ultraviolet radiation source is under operation without being in the direct field of irradiation of the ultraviolet radiation source and thus the ultraviolet radiation source does not need to be unnecessarily switched off.

With reference to the terms "downstream" and "upstream" it is understood that the respective term refers to a relative direction along, for example, an optical axis, wherein the relative direction relates to the direction of the ultraviolet radiation from the ultraviolet radiation source. Accordingly, downstream refers to the direction of the ultraviolet radiation from the ultraviolet radiation source and upstream refers to the opposite direction of the ultraviolet radiation from the ultraviolet radiation source. For example, when the ultraviolet radiation source is arranged upstream the optical system the radiation from the ultraviolet radiation source pass the optical system subsequent of leaving the ultraviolet radiation source. When the optical sensor is arranged upstream the optical system, the optical sensor may thus be arranged in substantially the same direction relative the optical system as the ultraviolet radiation source. For example, the optical sensor could be arranged in front of the ultraviolet radiation source, partly covering the field of irradiation and/or adjacent to the ultraviolet radiation source such that the optical sensor is arranged to the side, any side, of the ultraviolet radiation source as seen in a direction downstream the ultraviolet radiation source.

The term "ultraviolet radiation" refers to electromagnetic radiation with wavelengths in the ultraviolet spectral range, *i.e.* wavelengths in a range between 100 nm and 400 nm.

To have the field of view of the optical sensor to fully cover the field of irradiation of the ultraviolet radiation source may be favorable since this ensures detecting the presence or motion of objects at least within the field of irradiation of the ultraviolet radiation source. In some examples, the field of irradiation of the ultraviolet radiation source may be 75 % to 100 % of the field of view of the optical sensor providing the field of irradiation from the ultraviolet radiation source to be slightly smaller up to matching with the field of view of the optical sensor. In other words, the field of view of the optical sensor may be substantially the same as or larger than the field of irradiation of the ultraviolet radiation source. In cases when the field of irradiation from the ultraviolet radiation source is less than 100 % of the field of view of the optical sensor this may provide a safety perimeter just outside the field of irradiation where the presence or motion of objects are detected but no ultraviolet radiation is present. The ultraviolet radiation source may be configured to emit electromagnetic radiation of a first spectral range and the optical sensor may be configured to detect electromagnetic radiation of a second spectral range, wherein the first spectral range and the second spectral range are different. This may be advantageous in providing less interference from the ultraviolet radiation source when the optical sensor is detecting motion or presence of objects since the spectral sensitivity range of the optical sensor differs from the wavelengths used to illuminate. For example, the optical sensor may be arranged to detect at least one of visible light and infrared radiation. The optical sensor may for example be a passive infrared sensor, or any other optical sensor suitable to detect infrared radiation and/or visible light.

Electromagnetic radiation with wavelengths in a range from 1 nm to 1 mm may also be referred to as "optical radiation".

The ultraviolet radiation source may be configured to emit radiation of a first spectral range comprising optical radiation with wavelengths in a range of 100 to 280 nm, which wavelength range has shown to effectively kill bacteria and/or viruses.

The skilled person realizes that the emitted optical radiation may have a statistical distribution (*e.g.* a normal distribution or a gaussian distribution may be used to estimate the radiation distribution from a radiation source). The emitted radiation may be referred to as a beam of radiation and thus the intensity of the ultraviolet radiation will typically be larger in the center of the beam than in the periphery of the beam. The intensity will typically wear off towards the sides. Thus, when referring to the field of irradiation above, this should be understood as the vast majority of the ultraviolet radiation from the ultraviolet radiation source, such as at least 85 % or 90 %, preferably more than 95 %. The remaining percentage of radiation will be arranged outside the field of illumination and parts thereof may also, in some cases, fall within the field of view of the sensor (*e.g.* when the field of irradiation is defined as being less than the field of view of the sensor).

Further, the skilled person realizes that the optical sensor may be sensitive to light according to a statistical distribution, partly dependent on the optics used for collecting the light in front of the optical sensor. Hence, the optical sensor may detect light, *e.g.* objects emitting or reflecting light, with greater sensitivity for light arranged directly in front of the optical sensor, *e.g.* light arranged along a normal to a sensor surface facing the light, compared to light arranged at an angle from the normal to the sensor surface. Accordingly, when referring to the field of view above, this should be understood as a range of angles from the normal to the sensor surface wherein the range of angles is defined such that the vast majority of the light detected by the sensor is covered, such as at least 90 % or at least 95 %.

The optical system may be configured to shape the field of irradiation of the ultraviolet radiation source and the field of view of the optical sensor differently. The optical sensor may be arranged with respect to the radiation source such that the field of irradiation of the ultraviolet radiation source falls within the field of view of the optical sensor downstream of the optical system. This may be advantageous in that the optical sensor may be arranged with respect to the ultraviolet radiation source and the optical system such that the field of irradiation of the ultraviolet radiation source and the field of view of the optical sensor after the optical system coincides, *i.e.* the effect in shaping of the field of view of the optical sensor and the field of irradiation of the ultraviolet radiation source may be taken into account when positioning the optical sensor, the ultraviolet radiation source and the optical system. Further, this may be advantageous in providing the ability to configure the optical system to correct for misalignment between the field of irradiation of the ultraviolet radiation source and the field of view of the optical sensor such that the field of irradiation of the ultraviolet radiation source falls within the field of view of the optical sensor downstream of the optical system.

The luminaire according to the first aspect of the invention may be used for performing an ultraviolet germicidal irradiation (UVGI) disinfection method.

A method for controlling a luminaire according to the first aspect of the invention comprises the steps of (i) detecting, using the optical sensor, the presence or motion of an object within a field of view of the optical sensor, and (ii) switching off the ultraviolet radiation source in response to the detected presence or motion of an object, wherein a field of irradiation of the ultraviolet radiation source falls within the field of view of the optical sensor.

The above method provides the same advantages as were discussed in relation to the first aspect of the invention. Accordingly, the harmful ultraviolet radiation from the ultraviolet radiation source will be interrupted as soon as someone enters the field of view of the optical sensor which may fully cover the field of irradiation of the ultraviolet radiation source, thus this may be advantageous in providing a compact and reliable solution where the ultraviolet radiation is switched off only when someone risk entering the field of irradiation of the ultraviolet radiation source. A further scope of applicability of the present invention will become apparent from the detailed description given below. However, it should be understood that the detailed description and specific examples, while indicating preferred embodiments of the invention, are given by way of illustration only, since various changes and modifications within the scope of the invention will become apparent to those skilled in the art from this detailed description.

It is to be understood that this invention is not limited to the particular component parts of the device described as such device may vary. It is also to be understood that the terminology used herein is for purpose of describing particular embodiments only, and is not intended to be limiting. It must be noted that, as used in the specification and the appended claim, the articles "a", "an", and "the" are intended to mean that there are one or more of the elements unless the context clearly dictates otherwise. Thus, for example, reference to "a radiation source" or "the radiation source" may include several devices, and the like. Furthermore, the words "comprising", "including", "containing" and similar wordings does not exclude other elements or steps.

### BRIEF DESCRIPTION OF THE DRAWINGS

The above and other aspects of the present invention will now be described in more detail, with reference to the appended drawings showing embodiments of the invention. The figures should not be considered limiting the invention to the specific embodiment; instead they are used for explaining and understanding the invention.
Fig. 1 illustrates a luminaire comprising an ultraviolet radiation source having a field of irradiation and an optical sensor having a field of view.
Fig. 2A illustrates a perspective view of a luminaire comprising an ultraviolet radiation source, an optical sensor, a control unit and an optical system.
Fig. 2B illustrates part of a luminaire as seen from a direction downstream the luminaire.
Fig. 3 illustrates a method for controlling a luminaire.

As illustrated in the figures, the sizes of layers and regions are exaggerated for illustrative purposes and, thus, are provided to illustrate the general structures of embodiments of the present invention. Like reference numerals refer to like elements throughout.

### DETAILED DESCRIPTION OF THE EMBODIMENTS

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled person.

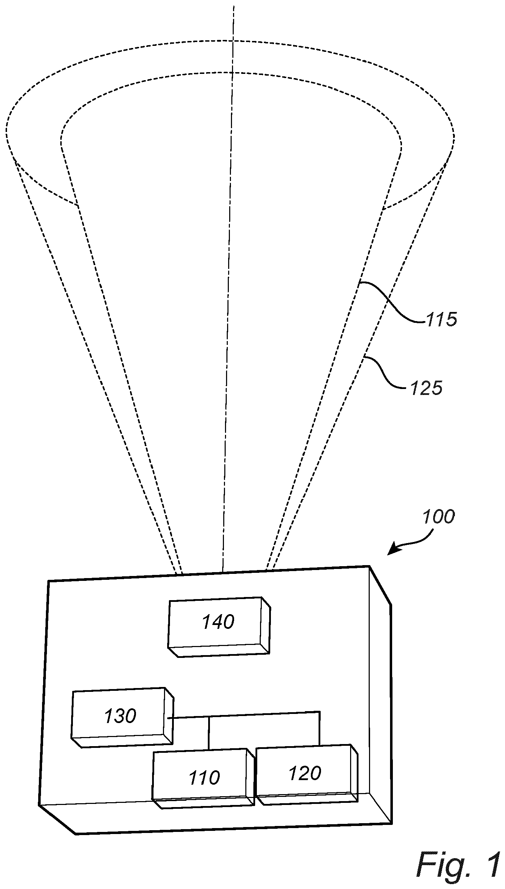

Fig. 1 schematically shows a luminaire 100 for emitting ultraviolet radiation. The luminaire 100 may be used for performing an ultraviolet germicidal irradiation (UVGI) disinfection method. However, the luminaire 100 may as well be used for any other type of treatment based on ultraviolet radiation. The luminaire 100 comprises an ultraviolet radiation source 110 having a field of irradiation 115, an optical sensor 120 having a field of view 125. The luminaire may further comprise a control unit 130 configured to control the ultraviolet radiation source 110. The control unit 130 may thus be connected to the ultraviolet radiation source. The optical sensor 120 may be configured to detect motion or presence of an object. The optical sensor 120 is preferably configured to detect motion or presence of a human or an animal. The optical sensor 120 may indicate the detected motion or presence of an object to the control unit 130. The control unit 130 may control the ultraviolet radiation source 110 based on the detected motion or presence of an object from the sensor.

For example, the optical sensor 120 may be configured to output a signal indicative of motion or presence of an object being detected. The control unit 130 may be configured to control the ultraviolet radiation source 110 based on the signal from the optical sensor 120. Hence, the control unit 130 may be configured to receive the signal from the optical sensor. The control unit 130 may be configured to switch off the ultraviolet radiation source 110 when the optical sensor 120 indicates detection of motion or presence of an object. In other words, control unit 130 may be configured to switch off the ultraviolet radiation source 110 upon the signal being indicative of motion or presence of an object.

The luminaire may further comprise an optical system 140. The optical system 140 may be an exit aperture to the luminaire. The optical system 140 may be arranged downstream the ultraviolet radiation source. The optical system 140 may further be configured to shape the field of irradiation 115 of the ultraviolet radiation source 110 and the field of view 125 of the optical sensor.

The ultraviolet radiation source 110 and the optical sensor 120 may both be located upstream of the optical system 140 such that the field of irradiation 115 of the ultraviolet radiation source 110 falls within the field of view 125 of the optical sensor 120 downstream of the optical system 140. The field of irradiation 115 may be at least 75 % of the field of view 125 of the optical sensor 120 in order for the field of irradiation 115 from the ultraviolet radiation source 110 to be of a reasonable size compared to the field of view 125 of the optical sensor. Hence, the field of view 125 of the optical sensor 120 outside the field of irradiation 115 may be limited in order to mitigate unnecessary deactivation of the ultraviolet radiation upon detection of movement or the presence of objects outside the field of irradiation 115 but within the field of view 125 of the optical sensor 120, *e.g.* when objects enter the field of view 125 but never enters the field of irradiation 115. The field of irradiation 115 of the ultraviolet radiation source 110 may for example be at least 75 % of the field of view 125 of the optical sensor 120. The field of irradiation 115 of the ultraviolet radiation source 110 may be less than 100 % of the field of view of the optical sensor 120. Alternatively, the field of irradiation 115 of the ultraviolet radiation source 110 may match the field of view 125 of the optical sensor 120, *i.e.* the field of irradiation 115 may be 100 % of the field of view 125 of the optical sensor 120. Accordingly, the field of irradiation 115 of the ultraviolet radiation source 110 may be 75 % to 100 % of the field of view 125 of the optical sensor 120.

The ultraviolet radiation source 110 may be configured to emit radiation of a first spectral range. The optical sensor 120 may be configured to detect optical radiation of a second spectral range. The first spectral range and the second spectral range may be different. For example, the optical sensor 120 may be configured to detect at least one of visible radiation and infrared radiation. Infrared radiation may be subdivided into IR-A (having wavelengths in a range of 800 to 1400 nm), IR-B (having wavelengths in a range of 1400 nm to 3,0 µm) and IR-C (having wavelengths in a range of 3,0 µm to 1 mm) according to DIN 5031. Accordingly, the optical sensor 120 may be configured to detect optical radiation within at least one of these wavelength ranges. Visible radiation refers to electromagnetic radiation having wavelengths in a range of 400 to 800 nm.

The optical sensor 120 may be a passive infrared sensor or any other sensor known in the art to detect at least one of visible radiation and infrared radiation.

Ultraviolet radiation may be divided into UV-A (having wavelengths in a range of 315 to 400 nm), UV-B (having wavelengths in a range of 280 to 315 nm) and UV-C (having wavelengths in a range of 100 to 280 nm). Of the wavelength ranges, the UV-C wavelength range has shown to be most effective in killing bacteria and viruses. UV-B may also provide a germicidal effect, but it is considered less effective than UV-C. Accordingly, the first spectral range may comprise ultraviolet radiation comprising wavelengths from at least one of the UV-B and UV-C wavelength ranges.

The optical system 140 may be configured to shape and/or modify the field of irradiation 115 of the ultraviolet radiation source 110 and the field of view 125 of the optical sensor 120 in substantially the same manner. Additionally, or optionally, the optical system 140 may be configured to shape the field of irradiation 115 of the ultraviolet radiation source 110 and the field of view 125 of the optical sensor 120 differently. This may be a result of for example the difference in wavelengths of the ultraviolet radiation and the optical radiation detected by the optical sensor 120. The optical system 140 may comprise optical elements that may affect the field of irradiation 115 of the ultraviolet radiation source 110 and field of view 125 of the optical sensor 120 slightly differently that may be caused by differences in spectral reflection and transmittance of materials in the optical elements.

Accordingly, when designing the luminaire 100, the optical system 140 may be configured to shape the field of irradiation 115 of the ultraviolet radiation source 110 and the field of view 125 of the optical sensor 120 such that the field of irradiation 115 of the ultraviolet radiation source 110 falls within the field of view 125 of the optical sensor 120 downstream of the optical system 140.

Accordingly, the optical sensor 120 may be arranged with respect to the radiation source 110 such that the field of irradiation 115 of the ultraviolet radiation source 110 falls within the field of view 125 of the optical sensor 120 downstream of the optical system 140.

It should be understood that the luminaire 100 could be used in various applications where a germicidal effect is beneficial, such as in medical facilities, hospitals, pharmacies and/or any other facility and/or application that could benefit from disinfecting surfaces and/or air.

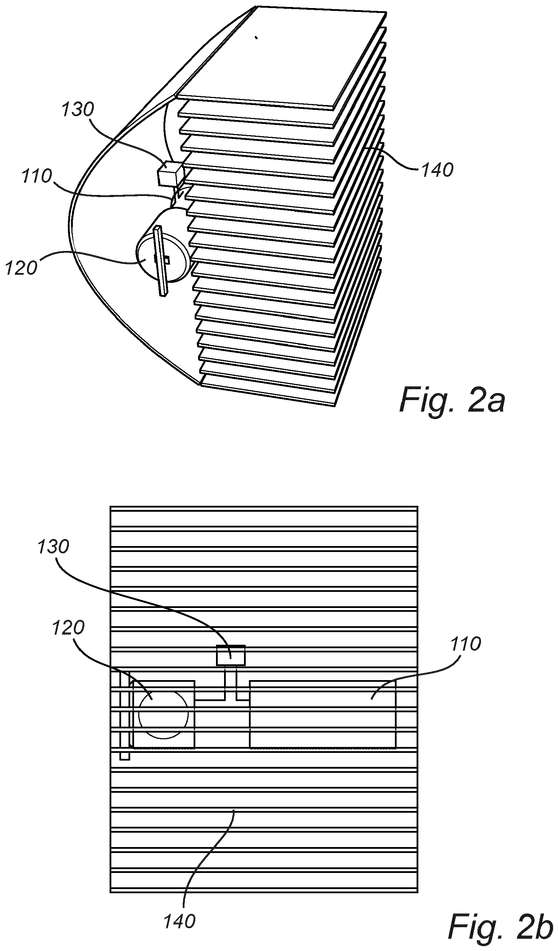

In Figs. 2a-2b the luminaire 100 is further illustrated and the relative position of the components therein are exemplified. In Fig. 2a, the optical system 140 is arranged downstream from the ultraviolet radiation source 110. The optical sensor 120 may be arranged adjacent to the ultraviolet radiation source. In some cases, it may be preferable to have the optical sensor 120 arranged as close to the ultraviolet radiation source 110 as possible. Accordingly, the ultraviolet radiation source 110 and the optical sensor 120 are both arranged upstream from the optical system 140. Further, the control unit 130 may be arranged adjacent to at least one of the ultraviolet radiation source 110 and the optical sensor 120. The control unit 130 may further be connected to the ultraviolet radiation source 110 and the optical sensor 120 in order to control the ultraviolet radiation source 110 based on for example motion or presence of an object detected by the optical sensor 120. The control unit 130 may be circuitry arranged adjacent to the optical sensor 120.

The optical system 140 may comprise a plurality of louvers or gratings. Alternatively, or additionally, the optical system 140 may comprise optical lenses made of a material that is permeable for ultraviolet radiation and/or reflectors, that could for example be used for beam shaping. The reflector may be reflective to optical radiation within at least one of ultraviolet wavelength ranges and the visible wavelength range.

By simulation the design of the system may be determined, *i.e.* how the optical sensor 120, the ultraviolet radiation source 110 and the optical system 140 may be arranged with respect to each other in order to have the field of irradiation 115 of the ultraviolet radiation source 110 to fall within the field of view 125 of the optical sensor 120. In the simulation the sensitivity distribution of the optical sensor 120 and the intensity distribution of the ultraviolet radiation source 110 may be taken into account. This may be especially advantageous in order to take into consideration differences in spectral refraction and reflections and transmittance due to differences in wavelength ranges. Further, during simulations the materials of the optical system 140 may be taken into consideration causing possible differences in spectral refraction and reflections and transmittance of the materials of the optical system 140.

The simulations may be advantageous in providing suitable positions for the optical sensor 120 relative the ultraviolet radiation source 110 and the optical system 140. Further, the simulations may be beneficial in providing fine tuning of the sensor's placement in order to optimize the desired sensitivity distribution.

In Fig. 2b, the ultraviolet radiation source 110, the optical sensor 120 and the control unit 130 are arranged behind the optical system 140 as seen in a direction along the ultraviolet radiation path. In Fig. 2b, the optical sensor 120 is arranged to the left of the ultraviolet radiation source 110 as seen from a direction along the ultraviolet radiation path. It should be understood that this is in no way limiting and the optical sensor 120 may be arranged on any side of the ultraviolet radiation source 110 as seen from a direction along the ultraviolet radiation path. The optical sensor 120 may alternatively be arranged in front of the ultraviolet radiation source 110 as seen from a direction along the ultraviolet radiation path. Hence, the optical sensor 120 may be at least partly covering the ultraviolet radiation source 110. In other words, the optical sensor 120 may be arranged between the ultraviolet radiation source 110 and the optical system 140.

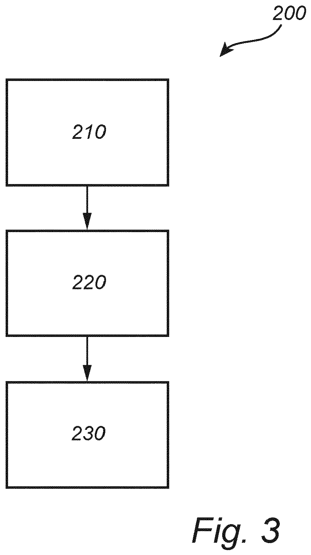

In Fig 3, a method 200 for controlling the luminaire 100 is illustrated. The method 200 comprises the step of detecting 210, using the optical sensor 120, the presence or motion of an object within a field of view 125 of the optical sensor 120 and the step of switching off 220 the ultraviolet radiation source 110 in response to the detected presence or motion of an object, wherein a field of irradiation 115 of the ultraviolet radiation source 110 falls within the field of view 125 of the optical sensor 120. The method 200 may further comprise outputting 230 a signal indicative of motion or presence of an object is detected, e.g. by transmitting a signal from the optical sensor 120 that may be received by the control unit 130. The control unit 130 may hence perform the step of switching off 220 the ultraviolet radiation source 110 in response to the received signal.

Additionally, variations to the disclosed embodiments can be understood and effected by the skilled person in practicing the claimed invention, from a study of the drawings, the disclosure, and the appended claims.

## Claims

1. A luminaire (100) for emitting ultraviolet radiation, the luminaire (100) comprising:
- an ultraviolet radiation source (110) having a field of irradiation (115),
- an optical sensor (120) having a field of view (125), the optical sensor (120) being configured to detect motion or presence of an object in the field of view (125) and to output a signal indicative of detected motion or presence of the object,
- a control unit (130) configured to control the ultraviolet radiation source (110) based on the signal from the optical sensor (120), the control unit (130) being connected to the ultraviolet radiation source (110) and being configured to switch off the ultraviolet radiation source (100) upon receipt of the signal indicative of detected motion or presence of the object; and
- an optical system (140),
**characterized in that** the ultraviolet radiation source (110) and the optical sensor (120) are both located upstream of the optical system (140),
wherein the optical system (140) is configured to shape the field of irradiation (115) of the ultraviolet radiation source (110) and the field of view (125) of the optical sensor (120) such that the field of irradiation (115) of the ultraviolet radiation source (110) falls within the field of view (125) of the optical sensor (120) downstream of the optical system (140).

2. The luminaire (100) according to claim 1, wherein the optical system (140) comprises a plurality of louvers or gratings.

3. The luminaire (100) according to any one of the preceding claims, wherein the field of irradiation (115) of the ultraviolet radiation source (110) is 75 % to 100 % of the field of view (125) of the optical sensor (120).

4. The luminaire (100) according to any one of the preceding claims, wherein the ultraviolet radiation source (110) is configured to emit radiation of a first spectral range and wherein the optical sensor is configured to detect radiation of a second spectral range, wherein the first spectral range and the second spectral range are different.

5. The luminaire (100) according to any one of the preceding claims, wherein the optical system (140) is configured to shape the field of irradiation (115) of the ultraviolet radiation source (110) and the field of view (125) of the optical sensor (120) differently.

6. The luminaire (100) according to any one of the preceding claims, wherein the optical sensor (120) is arranged to detect at least one of visible light and infrared radiation.

7. The luminaire (100) according to any one of the preceding claims, wherein the optical sensor (120) is a passive infrared sensor.

8. The luminaire (100) according to any one of the preceding claims, wherein the ultraviolet radiation source (110) is configured to emit radiation of a first spectral range, and wherein the first spectral range comprises radiation with wavelengths in a range of 100 to 280 nm.

## Patentansprüche

1. Leuchte (100) zum Emittieren von Ultraviolettstrahlung, die Leuchte (100) umfassend:
- eine Ultraviolettstrahlungsquelle (110), die ein Bestrahlungsfeld (115) aufweist,
- einen optischen Sensor (120), der ein Sichtfeld (125) aufweist, wobei der optische Sensor (120) konfiguriert ist, um eine Bewegung oder die Anwesenheit eines Objekts in dem Sichtfeld (125) zu erkennen und ein Signal auszugeben, das für eine erkannte Bewegung oder Anwesenheit des Objekts bezeichnend ist,
- eine Steuereinheit (130), die konfiguriert ist, um die Ultraviolettstrahlungsquelle (110) basierend auf dem Signal von dem optischen Sensor (120) zu steuern, wobei die Steuereinheit (130) mit der Ultraviolettstrahlungsquelle (110) verbunden ist und konfiguriert ist, um die Ultraviolettstrahlungsquelle (100) nach Empfang des Signals, das für eine erkannte Bewegung oder Anwesenheit des Objekts bezeichnend ist, auszuschalten; und
- ein optisches System (140),
**dadurch gekennzeichnet, dass** sich die Ultraviolettstrahlungsquelle (110) und der optische Sensor (120) beide dem optischen System (140) vorgeschaltet befinden,
wobei das optische System (140) konfiguriert ist, um das Bestrahlungsfeld (115) der Ultraviolettstrahlungsquelle (110) und das Sichtfeld (125) des optischen Sensors (120) derart zu formen, dass das Bestrahlungsfeld (115) der Ultraviolettstrahlungsquelle (110) in das Sichtfeld (125) des optischen Sensors (120), das dem optischen System (140) nachgeschaltet ist, fällt.

2. Leuchte (100) nach Anspruch 1, wobei das optische System (140) eine Vielzahl von Lamellen oder Gittern umfasst.

3. Leuchte (100) nach einem der vorstehenden Ansprüche, wobei das Bestrahlungsfeld (115) der Ultraviolettstrahlungsquelle (110) 75 % bis 100 % des Sichtfelds (125) des optischen Sensors (120) beträgt.

4. Leuchte (100) nach einem der vorstehenden Ansprüche, wobei die Ultraviolettstrahlungsquelle (110) konfiguriert ist, um Strahlung eines ersten Spektralbereichs zu emittieren, und wobei der optische Sensor konfiguriert ist, um Strahlung eines zweiten Spektralbereichs zu erkennen, wobei der erste Spektralbereich und der zweite Spektralbereich unterschiedlich sind.

5. Leuchte (100) nach einem der vorstehenden Ansprüche, wobei das optische System (140) konfiguriert ist, um das Bestrahlungsfeld (115) der Ultraviolettstrahlungsquelle (110) und das Sichtfeld (125) des optischen Sensors (120) unterschiedlich zu formen.

6. Leuchte (100) nach einem der vorstehenden Ansprüche, wobei der optische Sensor (120) angeordnet ist, um mindestens sichtbares Licht und Infrarotstrahlung zu erkennen.

7. Leuchte (100) nach einem der vorstehenden Ansprüche, wobei der optische Sensor (120) ein passiver Infrarotsensor ist.

8. Leuchte (100) nach einem der vorstehenden Ansprüche, wobei die Ultraviolettstrahlungsquelle (110) konfiguriert ist, um Strahlung eines ersten Spektralbereichs zu emittieren, und wobei der erste Spektralbereich Strahlung mit Wellenlängen in einem Bereich von 100 bis 280 nm umfasst.

## Revendications

1. Luminaire (100) pour émettre un rayonnement ultraviolet, le luminaire (100) comprenant :
- une source de rayonnement ultraviolet (110) ayant un champ d'irradiation (115),
- un capteur optique (120) ayant un champ de vision (125), le capteur optique (120) étant configuré pour détecter un mouvement ou une présence d'un objet dans le champ de vision (125) et pour produire un signal indiquant un mouvement détecté ou une présence de l'objet,
- une unité de commande (130) configurée pour commander la source de rayonnement ultraviolet (110) sur la base du signal provenant du capteur optique (120), l'unité de commande (130) étant connectée à la source de rayonnement ultraviolet (110) et configurée pour éteindre la source de rayonnement ultraviolet (100) à la réception du signal indiquant la détection un mouvement ou une présence de l'objet ; et
- un système optique (140),
**caractérisé en ce que** la source de rayonnement ultraviolet (110) et le capteur optique (120) sont tous deux situés en amont du système optique (140),
dans lequel le système optique (140) est configuré pour modeler le champ d'irradiation (115) de la source de rayonnement ultraviolet (110) et le champ de vision (125) du capteur optique (120) de telle sorte que le champ d'irradiation (115) de la source de rayonnement ultraviolet (110) tombe dans le champ de vision (125) du capteur optique (120) en aval du système optique (140).

2. Luminaire (100) selon la revendication 1, dans lequel le système optique (140) comprend une pluralité de lamelles ou de grilles.

3. Luminaire (100) selon l'une quelconque des revendications précédentes, dans lequel le champ d'irradiation (115) de la source de rayonnement ultraviolet (110) est compris entre 75 % et 100 % du champ de vision (125) du capteur optique (120).

4. Luminaire (100) selon l'une quelconque des revendications précédentes, dans lequel la source de rayonnement ultraviolet (110) est configurée pour émettre un rayonnement d'une première gamme spectrale et dans lequel le capteur optique est configuré pour détecter un rayonnement d'une seconde gamme spectrale, dans lequel la première gamme spectrale et la seconde gamme spectrale sont différentes.

5. Luminaire (100) selon l'une quelconque des revendications précédentes, dans lequel le système optique (140) est configuré pour modeler différemment le champ d'irradiation (115) de la source de rayonnement ultraviolet (110) et le champ de vision (125) du capteur optique (120).

6. Luminaire (100) selon l'une quelconque des revendications précédentes, dans lequel le capteur optique (120) est agencé pour détecter au moins l'un parmi une lumière visible et un rayonnement infrarouge.

7. Luminaire (100) selon l'une quelconque des revendications précédentes, dans lequel le capteur optique (120) est un capteur infrarouge passif.

8. Luminaire (100) selon l'une quelconque des revendications précédentes, dans lequel la source de rayonnement ultraviolet (110) est configurée pour émettre un rayonnement d'une première gamme spectrale, et dans lequel la première gamme spectrale comprend un rayonnement avec des longueurs d'onde dans une gamme de 100 à 280 nm.
